# EUROPÄISCHE PATENTANMELDUNG

(11) **EP 1 334 735 A1**
(43) Veröffentlichungstag der Anmeldung: **13.08.2003**
(21) Anmeldenummer: 03001485.6
(22) Anmeldetag: 23.01.2003
(51) Int. Cl.: A61L 9/012, A61L 9/014, A61L 9/04, B01D 53/02, A61L 9/16

(54) **Verfahren zum Desodorieren von Gebäuderäumen**

(30) Priorität: 29.01.2002 DE 10203339; 14.03.2002 DE 10211165
(71) Anmelder: Air & D-Sarl, 67190 Rosheim (FR)
(72) Erfinder: Wuest, Robert, 67190 Rosheim (FR)
(74) Vertreter: Grussdorf, Jürgen, Dr.

(57) **Zusammenfassung**

Die Erfindung betrifft ein Verfahren und eine Vorrichtung zum Desodorieren bzw. zum Beduften von Gebäuderäumen durch Behandeln der Raumluft mit aktiven Agentien, die mit den in der Luft enthaltenen übelriechenden Substanzen reagieren oder diese maskieren und/oder den Raum beduften.
Die aktiven Agentien sind in einer Matrix aus einem vernetzten, hydrophile Gruppen enthaltenden Polymeren verteilt und bilden mit diesem zusammen eine schwammartige Masse, aus der die aktiven Agentien langsam freigesetzt werden und verdunsten.

## Beschreibung

Die Erfindung betrifft ein Verfahren zum Desodorieren bzw. zum Beduften von Gebäuderäumen durch Behandeln der Raumluft mit aktiven Agentien, die mit den in der Luft enthaltenen übelriechenden Substanzen reagieren oder diese maskieren und/oder den Raum beduften.

In Räumen von Gebäuden, in denen sich eine große Anzahl an Menschen aufhalten, herrscht häufig schlechte Luft, die von Körperausdünstungen, Essensgeruch und Tabakrauch herrührt. Insbesondere in Hotelzimmern, die mit 20 bis 30 m² relativ klein und deren Fenster oft nicht zu öffnen sind, sowie in Großküchen und in Toiletten von Restaurants ist dieses Problem besonders gravierend. Es besteht daher das Bedürfnis nach einer effizienten und schnell wirkenden Beseitigung oder zumindest starker Verminderung dieser Gerüche.

Die daraus resultierende Aufgabe wird durch das erfindungsgemäße Verfahren gelöst. Gegenstand der Erfindung ist demzufolge ein Verfahren zum Desodorieren bzw. zum Beduften von Gebäuderäumen durch Behandeln der Raumluft mit aktiven Agentien, die mit den in der Luft enthaltenen übelriechenden Substanzen reagieren oder diese maskieren und/oder den Raum beduften, wobei die aktiven Agentien in einer Matrix aus einem vernetzten, hydrophile Gruppen enthaltenden Polymeren verteilt sind und die mit diesem zusammen eine schwammartige Masse bilden, aus der die aktiven Agentien langsam freigesetzt werden und verdunsten.

Gebäuderäume im Sinne der Erfindung umfassen z .B. Zimmer, Aufenthaltsräume, Hallen, Flure, Küchen und Toiletten von Krankenhäusern, Altersheimen, Kasinos, Bürogebäuden, Einkaufszentren, Parkhäusern und insbesondere von Hotels und Restaurants.

Geeignete Matrix-Polymere sind vernetzte maleinisierte oder epoxidierte Polymere und vernetzte (Meth-)Acrylat-Polymere.

Bevorzugt sind Kondensationsprodukte aus einem maleinisierten oder epoxidierten Polymeren und einem Vernetzer, vorzugsweise einem Polyamin. Geeignete Polymere sind Umsetzungsprodukte aus einem Polydien, z. B. Polybutadien, Polyoctadien und Soyabohnenöl, mit Maleinäureanhydrid, ferner Copolymere von Olefinen, wie Ethylen, mit Maleinsäureanhydrid, sowie epoxidiertes Polybutadien. Bevorzugte Vernetzer sind Polyamine, insbesondere Polyoxypropylendiamin und Polyoxypropylentriamin. Daneben sind auch Harnstoff, Polyethylenimin sowie Triethylenglykol als Vernetzer geeignet. Die Vernetzungsreaktion erfolgt vorzugsweise in alkoholischer Lösung, z. B. in Dipropylenglykol, bei erhöhter Temperatur. Als hydrophile Gruppen wirken insbesondere die von den Polyoxyalkylenpolyaminen stammenden -CRH-O- Gruppen, daneben auch die Maleinsäureanhydrid- und Carboxylgruppen oder Epoxidgruppen bzw. die -NR-CO- Gruppen des vernetzten Polymeren.

Eine andere Klasse von vernetzten Polymeren sind Copolymerisate von monofunktionellen (Meth-)Acrylat- Monomeren, z. B. Hydroxyethylacrylat oder Poly(propylenoxid)(ethylenoxid)monomethacrylat, mit einem polyfunktionellen (Meth-)Acrylat-Monomeren, z. B. Ethylenglykoldimethacrylat oder Polyethylenglykol-400- dimethacrylat. Die Herstellung der vernetzten (Meth-)Acrylat-Polymeren erfolgt durch radikalische Copolymerisation der Monomeren.

In beiden Fällen sind die vernetzten Polymeren in der Lage, Flüssigkeiten und Gase, z. B. die aktiven Agentien, zu absorbieren, wobei sich eine offenzellige Schwammstruktur ausbildet. Das vernetzte Polymere weist ein räumliches Netzwerk mit Poren auf, in dem die flüchtigen Fremdsubstanzen aufgesaugt und absorbiert werden können, so dass das Polymere wie ein Schwamm anquillt. Im angequollenen Zustand besteht das dreidimensionale Netzwerk aus Elementarzellen die im Mittel ein Volumen von 1 bis 1000 nm³, vorzugsweise von 3 bis 200 nm³, aufweisen.

Das vernetzte Polymere ist erfindungsgemäß mit einem aktiven Agens beladen und bildet mit diesem eine schwammartige Masse. Das aktive Agens wird daraus langsam freigesetzt und kann dann den Raum beduften und/oder mit in den Räumen vorhandenen übelriechenden Substanzen, z. B. Aminen, Ammoniak und Schwefelverbindungen reagieren, diese reduzieren oder maskieren. Die aktiven Agentien sind meist flüssige Aldehyde, Ketone, Alkohole, Terpene oder Ester, beispielsweise Vanillin, Eugenol, Thymol, Geraniol, Kampferöl, Citronellol, Linanol, Menthol, Cumarin, Citral, Alpha- Pinen, Nerylacetat, Linalylacetat, Butylhydroxytoluol, Salicylsäurebenzylester, C₇- bis C₁₂- Aldehyde, ferner natürliche ölige Essenzen, wie Harzöle, sowie Parfüme und Mischungen der genannten Substanzen. Neben eigentlichen chemischen Reaktionen, z. B. zwischen Ammoniak und Aldehyden, kann es auch zu Bindungen durch elektrostatische und van der Waals'sche Krafte kommen, wodurch die Geruchwahrnehmbarkeit zumindest herabgesetzt wird.

Bevorzugt werden die aktiven Agentien bei der Herstellung der vernetzten Polymeren durch Kondensation bzw. Polymerisation zugesetzt, man kann aber auch das vernetzte Polymere mit den aktiven Agentien tränken und damit anquellen. Das aktive Agens sollte in der schwammartigen Masse in Mengen von 10 bis 90 Gew.-%, vorzugsweise von 40 bis 80 Gew.-% enthalten sein.

Die schwammartige Masse kann neben der Polymermatrix und den aktiven Agentien noch weitere Zusatzstoffe, z. B. 1 bis 20 Gew.-% Flammschutzmittel, wie Zucker, Bromverbindungen oder Azodicarbonamid, 1 bis 8 Gew.-% Wasser, sowie Pulver zur Verminderung des Verbackens und Sublimationshilfsmittel enthalten.

Wesentlich ist, dass das aktive Agens mit dem zur Anwendung kommenden vernetzten Polymeren so abgestimmt ist, dass es nur sehr langsam und während des Wirkungszeitraums gleichmäßig aus der schwammartigen Masse freigesetzt wird und seine Wirkung mindestens drei Tage, vorzugsweise mindestens eine Woche und insbesondere mehr als einen Monat lang beibehält. Das freigesetzte aktive Agens kann mit den übelriechenden Substanzen in der Gasphase reagieren und/oder den üblen Geruch überdecken, wobei der Geruch im Idealfall unter die Wahrnehmungsgrenze gedrückt wird. Darüberhinaus ist die schwammartige Masse in der Lage, gasförmige übelriechende Substanzen zu absorbieren und dadurch aus der Luft zu entfernen. Wenn das aktive Agens als Duftstoff oder Parfüm wirkt, kann damit der Raum beduftet werden. Beide Wirkungen können sich auch überlagern.

Die schwammartige Masse, welche die aktiven Agentien enthält, kann in Form von Kugeln, Spänen oder Granulat zur Anwendung kommen. Sie wird jedoch bevorzugt in Form von Krümeln, Platten oder Steifen, vorzugsweise mit einer Dicke von 0,2 bis 5 cm, insbesondere von 0,5 bis 3 cm, eingesetzt. Die schwammartige Masse kann als Krümel, Späne, Granulat oder Streifen in Hülsen eingelegt werden, die an ihrem Umfang eine Vielzahl von Löchern aufweisen. Bevorzugt wird sie aber als Streifen oder Platte auf Gitter oder auf Netze aufgelegt. Besonders günstig ist es, dabei mehrere Gitter übereinander oder nebeneinander auf einem Gestell anzuordnen und dieses in einen Ventilationskasten einzubauen, der an der Belüftung des Gebäuderaums angebracht ist. Dort bewirkt die angesaugte Frischluft die Verdunstung der aktiven Agentien aus der schwammartigen Masse. In Räumen mit Zwangsbelüftung können die Behältnisse mit der erfindungsgemäßen schwammartigen Masse direkt vor dem Lüftungsgitter angebracht werden, sonst über Eingangstüren, Kippfenstern oder Oberlichtern. Bei Gebäuderäumen mit starker Geruchsentwicklung, z. B. bei Restaurantküchen und Toilettenanlagen, führt die ausgeblasene Abluft oft zu erheblicher Belästigung der Umgebung. Diese kann vermindert werden, wenn die erfindungsgemäße schwammartige Masse in den Abluftkamin eingelagert wird. Aufgrund der lang anhaltenden Wirkung der erfindungsgemäßen Einsätze muss die schwammartige Masse nur alle zwei bis zwölf Wochen ausgewechselt werden.

### Beispiel 1

21 g maleinisiertes Polybutadien (Umsetzungsprodukt von flüssigem Polybutadien mit Maleinsäureanhydrid - LITHENE der Fa. Revertex) wurden mit 79 g einer Mischung von öligen Essenzen als aktives Agens bei 45° C vermischt (Mischung A). 94 g des aktiven Agens und 7,5 g Polyoxypropylentriamin (MG 400) wurden vermischt (Mischung B). Die Mischungen A und B wurden zusammengerührt.
Die erhaltene schwammartige Masse wurde in Platten von 1 cm Dicke, 15 cm Breite und 20 cm Länge geschnitten und auf ein Metallgitter aufgelegt. Mehrere Gitter wurden übereinander in einem Gestell angeordnet. Dieses Gestell wurde vor dem Lufteintrittsgitter eines Hotelzimmers angebracht, wo es über mehr als einen Monat hinweg seine desodorierende Wirkung entfaltete.

### Beispiel 2

In einem geschlossenen Gefäß wurden 4 g Poly(propylenoxid)(ethylenoxid)-Monomethylacrylat mit 0,5 g Polyethylenglykol-400-Dimethacrylat vermischt und 100 Mikroliter 30%-iges Wasserstoffperoxid wurden injiziert. Die Mischung wurde 30 min. lang in einem Ultraschallbad evakuiert. Dann wurde die Mischung in ein offenes, flaches Gefäß geleert und dort bei 10° C 5 min. lang mit UV-Licht (290 bis 400 nm, 40 mW/cm²) in einem Abstand von 5 cm bestrahlt.
Das erhaltene vernetzte Polymere wurde mit Salicylsäurebenzylester als aktivem Agens getränkt. Die erhaltene schwammartige Masse wurde wie in Beispiel 1 weiterverarbeitet.

## Patentansprüche

1. Verfahren zum Desodorieren bzw. zum Beduften von Gebäuderäumen durch Behandeln der Raumluft mit aktiven Agentien, welche mit den in der Luft enthaltenen übelriechenden Substanzen reagieren oder diese maskieren und/oder den Raum beduften, **dadurch gekennzeichnet, dass** die aktiven Agentien in einer Matrix aus einem vernetzten, hydrophile Gruppen enthaltenden Polymeren verteilt sind und mit diesem zusammen eine schwammartige Masse bilden, aus der die aktiven Agentien langsam freigesetzt werden und verdunsten.

2. Verfahren nach Anspruch 1, **dadurch gekennzeichnet, dass** das vernetzte Polymere ein Kondensationsprodukt aus einem maleinisierten oder epoxidierten Polymeren und einem Vernetzer, vorzugsweise einem Polyamin, ist.

3. Verfahren nach Anspruch 1, **dadurch gekennzeichnet, dass** das vernetzte Polymere ein Copolymerisat aus einem monofunktionellen (Meth-)Acrylat-Monomeren und einem polyfunktionellen (Meth-)Acrylat-Monomeren ist.

4. Verfahren nach Anspruch 1, **dadurch gekennzeichnet, dass** die aktiven Agentien über einen Zeitraum von mindestens drei Tagen hinweg aus der schwammartigen Masse freigesetzt werden.

5. Verfahren nach Anspruch 1, **dadurch gekennzeichnet, dass** die aktiven Agentien in Mengen von 10 bis 90 Gew.-% in der schwammartigen Masse enthalten sind.

6. Verfahren nach Anspruch 1, **dadurch gekennzeichnet, dass** die aktiven Agentien Aldehyde, Ketone, Alkohole, Ester, Terpene oder natürliche ölige Essenzen, bzw. Duftstoffe oder Parfüme sind.

7. Verfahren nach Anspruch 5, **dadurch gekennzeichnet, dass** die schwammartige Masse zusätzlich Flammschutzmittel, Sublimationshilfsmittel und/oder Wasser enthält.

8. Verfahren nach Anspruch 1, **dadurch gekennzeichnet, dass** die schwammartige Masse, welche die aktiven Agentien enthält, in Form von Krümeln, Platten oder Streifen mit einer Dicke von 0,2 bis 5 cm eingesetzt wird.

9. Verfahren nach Anspruch 8, **dadurch gekennzeichnet, dass** die Krümel, Platten oder Streifen auf Gitter oder Netzen aufgelegt sind.

10. Verfahren nach Anspruch 9, **dadurch gekennzeichnet, dass** mehrere Gitter übereinander oder nebeneinander auf einem Gestell angeordnet sind.

11. Verfahren nach Anspruch 8, **dadurch gekennzeichnet, dass** die Gitter oder Netze, die gegebenenfalls in einem Gestell angeordnet sind, in dem Luftschacht des Gebäuderaums eingebracht sind.

12. Verfahren nach Anspruch 1 zum Desodorieren von Zimmern, Aufenthaltsräumen, Hallen, Fluren, Küchen und Toiletten von Krankenhäusern, Altersheimen, Kasinos, Bürogebäuden, Einkaufszentren, Parkhäusern und insbesondere von Hotels und Restaurants.

13. Vorrichtung zur Durchführung des Verfahrens nach Anspruch 1, umfassend ein Gestell, auf dem mehrere Gitter übereinander oder nebeneinander angeordnet sind, auf denen Krümel, Streifen oder Platten aus einer schwammartigen Masse aufgelegt sind, welche desodorierende bzw. beduftende Agentien enthält.
